# EUROPEAN PATENT SPECIFICATION

(11) **EP 3 583 255 B1**
(45) Date of publication and mention of the grant of the patent: **23.08.2023**
(21) Application number: 18705636.1
(22) Date of filing: 15.02.2018
(51) Int. Cl.: D01F 4/00, D01D 10/02, D04H 1/30, D01F 2/00, D04H 1/4258, D04H 1/4266, D01D 5/26, D01D 5/04, D01D 7/00

(54) **METHOD AND APPARATUS FOR MANUFACTURING A STAPLE FIBER BASED ON NATURAL PROTEIN FIBER AND A RAW WOOL BASED ON THE STAPLE FIBER.**
VERFAHREN UND VORRICHTUNG ZUR HERSTELLUNG EINER STAPELFASER AUF DER BASIS VON NATÜRLICHER PROTEINFASER UND EINE ROHWOLLE AUF DER BASIS DER STAPELFASER.
PROCÉDÉ ET APPAREIL PERMETTANT DE FABRIQUER UNE FIBRE COUPÉE À BASE DE FIBRE DE PROTÉINE NATURELLE ET UNE LAINE BRUTE À BASE DE FIBRE COUPÉE.

(30) Priority: 15.02.2017 FI 20175134
(43) Date of publication of application: 25.12.2019
(73) Proprietor: Ecco Sko A/S, 6261 Bredebro (DK); Spinnova Oy, 40800 Vaajakoski (FI)
(72) Inventor: LIUKKONEN, Johanna, 40530 Jyväskylä (FI); HAAVISTO, Sanna, 40100 Jyväskylä (FI); SELENIUS, Pasi, 41400 Lievestuore (FI); SALMELA, Juha, 41340 Laukaa (FI); PORANEN, Janne, 40950 Muurame (FI); SALMINEN, Arto, 40420 Jyskä (FI); MYLLYS, Markko, 40700 Jyväskylä (FI); VENTO, Pia, 40800 Vaajakoski (FI); BJÖRKLUND, Karri, 40700 Jyväskylä (FI); GØGSIG, Thomas, 6261 Bredebro (DK); PETRUSIC, Stojanka, 6261 Bredebro (DK)
(74) Representative: Patentgruppen A/S
(86) International application number: PCT/EP2018/053849
(87) International publication number: WO 2018/149950

(56) References cited:
- CN-A- 102 174 720
- CN-A- 102 560 735
- US-A- 3 110 549
- US-A- 3 556 969
- US-A1- 2007 225 631

## Description

### Technical field

The present application relates to a method of manufacturing staple fiber based on natural protein fiber, an apparatus for manufacturing a staple fiber, a raw wool comprising staple fibers based on natural protein fiber, a fibrous yarn and a surface for use in an apparatus for manufacturing of staple fibers.

### Background

Products, like yarn and fabric, may be made of natural or synthetic starting material. Synthetic starting material may pose negative effects on recyclability or disposing phases of products.

A further challenge in relation to such materials is that synthetic starting materials are very attractive in relation to pricing but very often seen as inferior in terms of desired properties when compared to applications based on natural fibers.

Natural starting material may also pose negative environmental effects during its growth or processing. Example of such natural starting material is cotton.

The document US 3,110,549 A discloses a method of preparing formed collagen, where a mixture of so-called native collagen fiber and solubilized collagen. The mixture of native collagen fiber and solubilized collagen may be formed in different ways into sheets, filaments or solid or hollow bodies.

### Summary

The invention relates to a method according to claim 1 for manufacturing a staple fiber based on natural protein fiber, the method comprising the steps of
- providing a protein suspension,
- the protein suspension comprising fibrils of said natural protein fiber,
- directing the protein suspension through a nozzle (520) onto a surface (500),
- drying the protein suspension on the surface to a dry concentration of at least 70% by weight for forming a protein based fiber,
- extracting the fiber from the surface and providing the staple fiber.

It should be noted that the process step of extracting the fiber from the surface may be performed before, at the same time or after the staple fiber has been defined, e.g. by cutting, etc. The extraction process may thus involve that the fibers are divided into lengths of staple fibers defined by the structure of surface upon which the protein suspension was deposited. An alternative method may also include that the suspension is deposited on the surface and then removed from the surface as a fibril and then cut into the desired length(s) thereby providing the staple fibers.

According to an advantageous embodiment of the invention, it is possible to reconstruct staple fiber suitable for manufacturing of yarn having surprisingly combined stretch and strength properties and where the reconstructed fiber or the produced yarn is based on natural protein fibers.

In particular, it is noted that the use of protein fibers, such as collagen may also benefit from other advantages such as reduced flammability, the latter being of great concern when the yarn or fiber is used in relation to fabrics.

It is also noted that the present method facilitates an attractive use of protein fibers for the yarn or the fibers used in the yarn which is much more efficient than known yarn or fiber manufacturing methods.

Thus, the present invention makes it possible to provide a protein raw material which is rich in the concentration of the desired protein fibers, but where the fiber lengths of the protein fibers in the raw material are of less concern due to the fact, that the effective lengths of the produced protein staple fibers are so to speak, made to order.

This is an advantage in particular in relation to natural collagen fibers, as the length of these will typically vary depending on where these are derived from, e.g. skin or muscles. A challenge is thus that the typical attractive sources may very well comprise protein fibers of a desired dimension when considering the final purpose of spinning these in yarn and optionally also in a high amount, but where extraction of the fibers will rip the desired protein fibers apart. According to the present invention it is therefore possible to apply a source rich of protein fiber not necessarily of the desired length and then reconstruct these protein fibers into fibers having a desired length, in this context referred to staple fibers.

A further advantage of this inventive method is that strength properties of a produced yarn may be increased if the individual staple fibers have a length which is higher than either the protein fiber length in the source of natural protein or the protein fiber length of the fiber when it has been extracted from the source of natural protein.

The present invention may possess the following advantages: protein fiber based raw wool with attractive tensile strength and stretch properties, utilization of hazardous waste products, such as collagen waste products in general or wet blue leather waste or wet white leather waste, for generation of new sustainable products as such protein or collagen sources may thus be reused.

It is also noted that yarn produced from the protein fibers according to the invention may possess significantly different properties when compared to e.g. cellulose. Such different properties may include stretch, which is important in relation to many types of fabrics depending on the final application.

A protein suspension comprises an aqueous suspension of water and protein fibril obtained from natural protein fibers. Additionally, the protein suspension may comprise at least one rheology modifier. The protein suspension is directed through a nozzle onto a surface for drying. The protein suspension is dried by removing water or solvent from the protein suspension. Thereby a reconstructed fiber is formed on the surface, for example on a belt or a cylinder surface.

The term reconstructed fiber in this context is used as a way of designating that a staple fiber is produced on the basis of a number of mechanically sub-divided protein fibrils. The staple fiber can therefore be regarded as based on natural protein fibers.

In a very advantageous embodiment of the invention the invention utilizes a protein based suspension for feeding to the nozzle. The protein suspension comprises an aqueous suspension of water and protein. Additionally, the protein suspension may comprise at least one rheology modifier. The protein suspension is directed through a nozzle onto a surface for drying. The protein suspension is dried by removing water from the protein suspension. Thereby a reconstructed fiber is formed on the surface, for example on a belt or a cylinder surface.

The reconstructed fiber is extracted from the surface. The reconstructed fiber may be shortened into staple fibers of a certain length in order to make raw wool. This may be implemented on the surface or after extracted from the surface. The fiber based raw wool comprises staple fibers of certain length. Staple fibers are arranged in random order in order to form a raw wool network comprising staple fibers of certain length. The natural fiber based raw wool comprises staple fibers in fluffy, airy, loose arrangement such that order and density of the staple fibers among the natural fiber based raw wool is uneven. The natural fiber based raw wool comprises inhomogeneous structure. A staple fiber based raw wool may be processed in order to provide yarn or non-woven material.

According to an aspect of the invention a method for manufacturing a natural fiber based raw wool according to claim 12 comprises providing a protein suspension including water and protein fibrils. The method further comprises directing the protein suspension through a nozzle onto a surface, drying the protein suspension on the surface for forming a reconstructed fiber, and extracting the reconstructed fiber from the surface in order to form a staple fibers. The process may thus established filament, which is then cut or somehow divided, or the process may create the final on the surface.

An apparatus for manufacturing a natural fiber based raw wool according to claim 9 of the invention a nozzle arranged to direct a protein aqueous suspension including protein fibrils onto a surface. The apparatus further comprises a dryer arranged to dry the protein suspension on the surface for forming a reconstructed fiber, and extractor arranged to extract the reconstructed fiber from the surface in order to form a reconstructed fiber based raw wool comprising staple fibers. The process may thus establish filament, which is then cut or somehow divided, or the process may create the final on the surface.

The surface for forming staple fibers from a protein suspension may comprise an aqueous suspension of protein fibrils. The surface comprises a radius of curvature of 0.25-4 m; and grooves aligned on the surface perpendicular to the direction of movement of the surface. Grooves may be placed at predetermined constant intervals.

An aspect of an invention relates to a fiber based raw wool manufactured according to previous manufacturing method and/or apparatus. A fiber based raw wool comprising staple fibers, wherein the staple fibers are comprised of protein fibrils interlocked by hydrogen bonds and the fiber based raw wool comprises non-oriented, entangled, fluffy network of staple fibers.

In an example not falling under the scope of the claims, a yarn is made of staple fiber wool. Another aspect of the invention relates to a non-woven material made of staple fiber based raw wool.

In an embodiment of the invention, the natural protein fibers comprise collagen fibers.

The natural protein fibers may comprise elastin fibers.

The natural protein fibers may comprise resilin fibers.

The natural protein fibers may comprise keratin fibers.

In an embodiment of the invention, the fibrils of said natural protein fiber are mechanically divided from a source of natural protein fibers, such as natural collagen fibers.

As understood within the scope of the invention, mechanically divided refers to a mechanical process where a base material, the protein fibers are mechanically broken, separated or subdivided into entities. These entities may comprise one or more of the protein fibrils in question. It is therefore understood, that the inventive reconstruction of a filament within the scope of the invention may be based on single fibrils or also on groups of coherent fibrils. This is also consistent with the claiming.

The desired length of the fibrils may vary depending on the application, but the fibril should typically be less than 1 mm or typically smaller. In relation to collagen, this would very often result in that the fibers of the base material, i.e. the natural protein fiber, are shortened significantly. This may to a certain degree be done mechanically, but the process may also include a further refining, including mechanical refining. Generally, it can be said that the protein fibrils are divided protein fibers and that the protein fibrils are mechanically divided into entities by removing bonds in the protein fibers that links the protein fibrils. The length of the mechanically separated protein fibrils, preferably the collagen fibrils may in some instances be shorter than the originating protein fibers. The protein fibrils are subsequently gathered into a reconstructed staple fiber and this staple fiber may typically be longer than the produced protein fibrils. According to the present invention, it is possible to produce a staple fiber almost at measure on the basis of the shortened protein fibrils. This is in particular an advantage in relation to reconstructed staple fibers based on collagen fibrils as these tend to apply very attractive properties not offered by other types of compatible synthetic fibers. But this combined with the fact that collagen a relatively low fiber strength may be partly compensated for when spinning a final yarn by using long reconstructed staple fibers based on collagen fibrils.

In an embodiment of the invention, the protein suspension exiting the nozzle comprises a shape of the nozzle outlet opening, optionally a round or an elliptic cross-sectional shape.

The drying may comprise drying the protein suspension on a surface via radiation, convection or conduction.

, The surface may be a hydrophobic external surface.

The method may include a step of applying additive that reduces surface and suspension contact angle hysteresis, e.g. oil or wax, to the surface.

, Chemical bonds between the protein fibrils may be provided during said drying.

In other words, chemical bonds may gather the protein fibrils.

The staple fiber may comprise a length of 6-80 mm, preferably 40-60 mm.

, The fibers extracted from the drying surface may be shortened in order to form staple fibers.

In an embodiment of the invention the reconstructed staple fibers are combined into a raw wool.

In the present context, raw wool basically has the meaning that the provided staple fibers are combined into a wool which to a certain degree may resemble a natural wool, but the main understanding is basically that the produced wool constitutes a base material which is ready for carding and spinning.

The raw wool may be processed to form yarn strand or non-woven material.

The raw wool may be carded in order to orient and entangle the fibers.

The raw wool comprising the reconstructed protein fibers may be processed into a pre-yarn and/or spun into a yarn.

The reconstructed staple fibers may be processed to the form of non-woven material.

In an embodiment of the invention, the protein suspension comprises fibrils of natural protein fibers and wherein the protein content of the suspension is more than 50% of the total weight of natural fiber in the suspension.

In an embodiment of the invention, the collagen suspension comprises collagen fibrils of naturals fibers and wherein the collagen content of the suspension is more than 50% of the total weight of natural fiber fibrils in the suspension.

In an embodiment of the invention, the protein suspension comprises fibrils of natural fibers and wherein the collagen protein content of the suspension is more than 60% of the total weight of natural fiber fibrils in the suspension.

The protein suspension used in the process comprises fibrils of natural fibers, and the collagen protein content of the suspension may be more than 70% of the total weight of natural fiber fibrils in the suspension.

The protein suspension used in the process comprises fibrils of natural fibers, and the collagen protein content of the suspension may be more than 80% of the total weight of natural fiber fibrils in the suspension.

The protein suspension used in the process comprises fibrils of natural fibers, and the collagen protein content of the suspension may be more than 90% of the total weight of natural fiber fibrils in the suspension.

The protein suspension used in the process comprises fibrils of natural fibers, and the collagen protein content of the suspension may be more than 95% of the total weight of natural fiber fibrils in the suspension.

In an embodiment of the invention, the protein suspension further comprises additive(s) including rheology modifier(s), binder(s), cation active reagent(s), crosslinking agent(s), dispersion agent(s), pigment(s), and/or other modifier(s)

The protein suspension may comprise additive(s), such as alginate, alginic acid, pectin, carrageenan, anionic polyacrylamide (APAM), cationic polyacrylamide (CPAM), polyethylene oxide (PEO), carboxymethyl cellulose (CMC), starch, enzymes (such as laccase, transglutaminase), polyphenols, glutaraldehyde, gelatine, casein, glucose, Tris-HCl, phosphate or a combination of such.

Wherein the additive in the protein suspension when dried promotes molecular bonding between the fibrils when the protein suspension is dried on the surface.

The invention relates in a further aspect to an apparatus for manufacturing a staple fiber based on natural protein fiber comprising
- a nozzle arranged to direct a protein suspension onto a surface,
- the protein suspension comprising fibrils of said natural protein fiber,
- a dryer arranged to dry the protein suspension on the surface for forming a fiber,
- an extractor arranged to extract the fiber from the surface and an arrangement to form the staple fiber.

In an embodiment of the invention, the protein suspension is directed to the surface and comprises a cross- sectional shape corresponding to a shape of a nozzle outlet opening, optionally a round or an elliptic cross-sectional shape.

The dryer may comprise at least one of a heating resistor, a radiator, a vaporizer or a blower.

The surface may comprise a hydrophobic external surface.

In an embodiment of the invention, the fiber comprises dry concentration of at least 70 wt-%.

In an embodiment of the invention, the fibrils are interlocked via chemical bonds.

The staple fibers may comprise lengths of 6-80 mm, preferably 30-70 mm.

The apparatus may comprise an arrangement for shortening the fiber extracted from the surface in order to form staple fiber based raw wool.

The surface may comprise a curved surface.

The surface may comprise a grooved or ridged surface.

The surface may be curved and the surface may comprise grooves or ridges.

The surface may comprise a radius of curvature of 0.25-4 m.

The surface may comprise grooves or ridges extending perpendicular to a direction of a relative movement between the nozzle(s) and the surface.

The grooves may comprise lengths, parallel with the surface, of 0.5-5 mm, preferably 2-3 mm; and/or depth, perpendicular to the surface, of 0.5-10 mm, preferably 2-3 mm.

The invention relates in a further aspect to a fiber based raw wool comprising staple fibers, wherein the staple fibers reconstructed on the basis of protein fibrils are mechanically subdivided from natural protein fibers, wherein the protein fibrils are interlocked by hydrogen bonds and the fiber based raw wool comprises unoriented, entangled, fluffy network of staple fibers.

According to an embodiment of the invention, the fiber based raw wool comprises staple fibers, wherein the fibrils of the staple fibers are comprised of collagen fibrils from natural fibers.

The fiber based raw wool comprises staple fibers, wherein the staple fibers may comprise fibrils of collagen and elastin.

The fiber based raw wool comprises staple fibers, wherein the staple fibers may comprise fibrils of collagen and resilin.

The fiber based raw wool comprises staple fibers, wherein the staple fibers may comprise fibrils of collagen and keratin.

The fiber based raw wool may be processable, cardable and/or spinnable in order to form a yarn and/or non-woven material.

The staple fibers may comprise lengths of 6-80 mm, preferably 40-60 mm.

The staple fiber yarn count may comprise 1-20 dtex; and/or the staple fiber diameter may comprise 15-70 µm; and/or the staple fiber tensile strength may comprise 15-25 cN/tex; and/or the elongation at break may be 5-15%.

The invention relates in a further aspect to a fibrous yarn made of staple fiber according to the method of claims 1-8 and/or to a fiber based raw wool according to claim 12and/or manufactured using the apparatus according to claims 9-11.

The yarn may comprise a yarn count of 5-200 tex; and/or tenacity of 5-15 cN/tex; and/or elongation at break of 10-30 %.

The surface may comprise a cylinder or a belt.

### Description of drawings

In the following embodiments of the invention are described with the accompanying figures of which
Figure 1 illustrates a method for manufacturing a fiber based raw wool according to an embodiment.
Figure 2 illustrates a method for manufacturing a fiber based raw wool according to an embodiment.
Figure 3 illustrates an apparatus for manufacturing a fiber based raw wool according to an embodiment.
Figure 4 illustrates a cylinder as an example of a surface according to an embodiment.
Figure 5 illustrates an apparatus for manufacturing a fiber based raw wool according to an embodiment.
Figure 6 illustrates a belt as an example of a surface according to an embodiment.
Figure 7 illustrates a staple fiber according to an embodiment of the invention.
Figure 8 illustrates a staple fiber according to an embodiment of the invention.
Figure 9 illustrates a fiber based raw wool.
Figure 10 illustrates a tensile strength graph.

### Description of embodiments

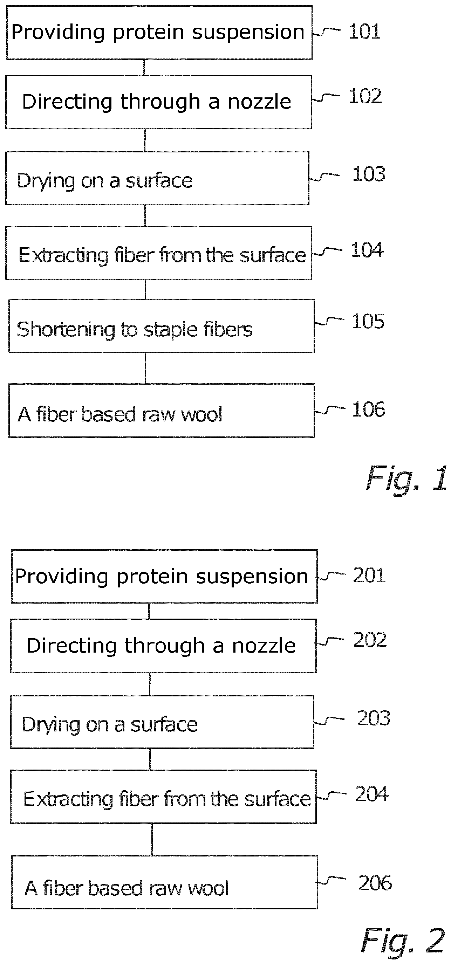

Figure 1 illustrates a method for manufacturing a natural fiber based raw wool according to an embodiment. A protein suspension is provided 101. The protein suspension comprises aqueous suspension of mechanically divided protein fibrils. In other words, the protein fibrils as such are to be regarded as natural fibril. The protein suspension may comprise water, divided protein fibrils and at least one rheology modifier. Fibrils of the protein suspension may originate from mechanically shortened protein fibers. In a preferred embodiment of the invention, the protein fibrils are collagen fibrils originating from tanned hide-based material. The collagen fibrils may thus originate from e.g. wet-blue, i.e. hides which has been tanned and where the collagen fibers used as a basis for the reconstructing of a collagen based filament has been pretreated e.g. by chromium salts according to conventional and well-known tanning methods.

The protein suspension is directed through a nozzle 102. The nozzle feeds the protein suspension to a surface. The surface may be a surface of a belt or of a cylinder. The protein suspension is dried on the surface 103. Drying removes water from the protein suspension. The dried protein suspension is arranged to form a reconstructed fiber on the surface. The reconstructed fiber may be arranged in a form of a continuous reconstructed fiber. The continuous reconstructed fiber is extracted from the surface 104. The reconstructed fiber extracted from the surface is cut or shortened in order to form staple fibers 105. The stable fibers are arranged to form an inhomogeneous network comprising fiber concentrations of varying density and orientation. The inhomogeneous fluffy material of staple fibers is called a natural fiber based raw wool 106.

The above process is described as a process using collagen fibers as a source for the manufacture of staple fibers. The process may be repeated with another protein fiber base, such as elastin or resilin.

Figure 2 illustrates a method for manufacturing a natural fiber based raw wool according to an embodiment. A protein suspension is provided 201. The protein suspension comprises aqueous suspension of divided protein fibrils. The protein suspension may comprise water, at least one rheology modifier and divided protein fibrils. Divided protein fibrils may originate from tanned hides, which has been mechanically subdivided into pulp. The protein suspension is directed through a nozzle 202. The nozzle feeds the protein suspension to a surface, for example on a surface of a belt or of a cylinder. The protein suspension is arranged to be shortened and dried on the surface 203. The dried and shortened protein suspension is arranged to form staple fibers. This is enabled by grooves (not shown) arranged on a surface. In following embodiments grooves refer to structures enabling a formation of staple fibers having a length defined by the distance between the groove. Those grooves, including grooves 401 and 601 may generally alternatively be made as ridges performing the same function, i.e. weakening strength of the reconstructed filament at positions where the filament may divide or break into the desired reconstructed staple fibers. The shortened fibers are extracted from the surface 204. The stable fibers are arranged to form an inhomogeneous network comprising fiber concentrations of varying density and orientation. The inhomogeneous fluffy material of staple fibers is called a natural fiber based raw wool 206.

A protein suspension could comprise collagen fibrils. Collagen fibrils are natural fibrils originating from shavings, leather waste, pulp and/or tanned hides including the hazardous waste material from the tanning process. The collagen fibrils are thus originating from different animals, typically skin. This may include wet blue and wet white shavings or leather shavings or tanned hide shavings.

Collagen may have macromolecular structure repeating amino acid sequences (GPX)n and glycine(G)-X-hydroxyproline(HYP). (GPX)n is the most common amino acid sequence in collagen. In the sequence X refers to any amino acid other than glycine, proline or hydroxyproline. The amino acid composition of collagen is atypical for proteins, particularly with respect to its high hydroxyproline content. Collagens are the most abundant proteins found in tissues of animals. Collagen is main structural protein in extracellular space in the connective tissues including tendons, ligaments and skin. There are several types of collagen. Type I (skin, tendon, bone), II (hyaline cartilage), III (reticulate), V (cell surfaces, hair, placenta) and XI (cartilage) being fibrillar. A single collagen molecule, tropocollagen, is used to make up larger collagen aggregates, such as fibrils. A collagen fibril is approximately 10 micrometers long and a diameter around 10 to 500nm. It is made up of three polypeptide strands (called alpha peptides), each of which has the conformation of a left-handed helix. These three left-handed helices are twisted together into a right- handed triple helix or "super helix", a cooperative quaternary structure stabilized by many hydrogen bonds. With type I collagen and possibly all fibrillar collagens, if not all collagens, each triple-helix associate into a right-handed super-super-coil referred to as the collagen microfibril. Each microfibril is interdigitated with its neighboring microfibrils.

If collagen is irreversibly partially hydrolyzed (e.g., by heat and chemicals), it is termed gelatin. Gelatin is modified protein.

Collagen fibrils may be in native form, which have not undergone any chemical modification. Natural collagen fibers and natural collagen fibrils may be non-regenerated. Thus, natural collagen fibers/fibrils have not undergone chemical regeneration or physical modification of the collagen polymer structure. It should nevertheless be noted that the collagen fibrils may have been chemically or at least physically affected when the source of collagen fibrils are tanned hides of animals.

Mechanical disintegration into protein fibrils from protein raw material, protein pulp, or refined pulp is carried out with suitable equipment such as a refiner, grinder, homogenizer, colloider, friction grinder, ultrasound sonicator, fluidizer such as microfluidizer, macrofluidizer or fluidizer-type homogenizer.

In particular and advantageously mechanical disintegration into collagen fibrils from collagen raw material, collagen pulp, or refined collagen pulp is carried out with suitable equipment such as a refiner, grinder, homogenizer, colloider, friction grinder, ultrasound sonicator, fluidizer such as microfluidizer, macrofluidizer or fluidizer-type homogenizer.

Protein fibers, such as collagen fibers may be isolated from any relevant protein containing raw material using chemical-, mechanical-, bio-, thermo-mechanical-, or chemi-thermo- mechanical pulping process. Mechanically shortened, divided or cut fibers may comprise chemically or physically modified derivative of collagen micro fibrils or fibril bundles.

A protein suspension may comprise 80-98 wt-% of water and 2-20 wt-% of protein. The protein suspension may comprise 85-98 wt-% of water and 2-15 wt% of protein. In addition, the protein suspension may comprise 0-5 wt-% of rheology modifier.

A collagen suspension may comprise 80-98 wt-% of water and 2-20 wt-% of collagen. The collagen suspension may comprise 85-98 wt-% of water and 2-15 wt% of collagen. In addition, the collagen suspension may comprise 0-5 wt-% of rheology modifier.

The mechanically divided protein fibril may be pure protein structures, or comprise chemically modified or chemically treated protein fibrils. Thus, the protein suspension comprises mechanically divided or shortened protein fibers. According to a preferred embodiment of the invention, the applied protein source is collagen.

Fiber product may include additives like rheology modifier(s), binder(s), cation active reagent(s), crosslinking agent(s), dispersion agent(s), pigment(s), and/or other modifier(s).

Fiber and staple fibers may comprise additives such as alginate, alginic acid, pectin, carrageenan, cationic polyacrylamide (CPAM), anionic polyacrylamide (APAM), cationic polyacrylamide (CPAM) i,cationic starch, chitosan, polyethylene oxide (PEO), carboxymethyl cellulose (CMC), starch, enzymes (such as laccase, transglutaminase), polyphenols, glutaraldehyde, gelatine, casein, glucose, Tris-HCl, phosphate or a combination of such.

Rheology modifier comprises a compound or agent arranged to modify the viscosity, yield stress and/or thixotropy of the suspension. Rheology modifier may comprise high molecular weight polymers. Rheology modifier is arranged to modify protein suspension rheology by adjusting gel strength and yield point of the protein suspension.

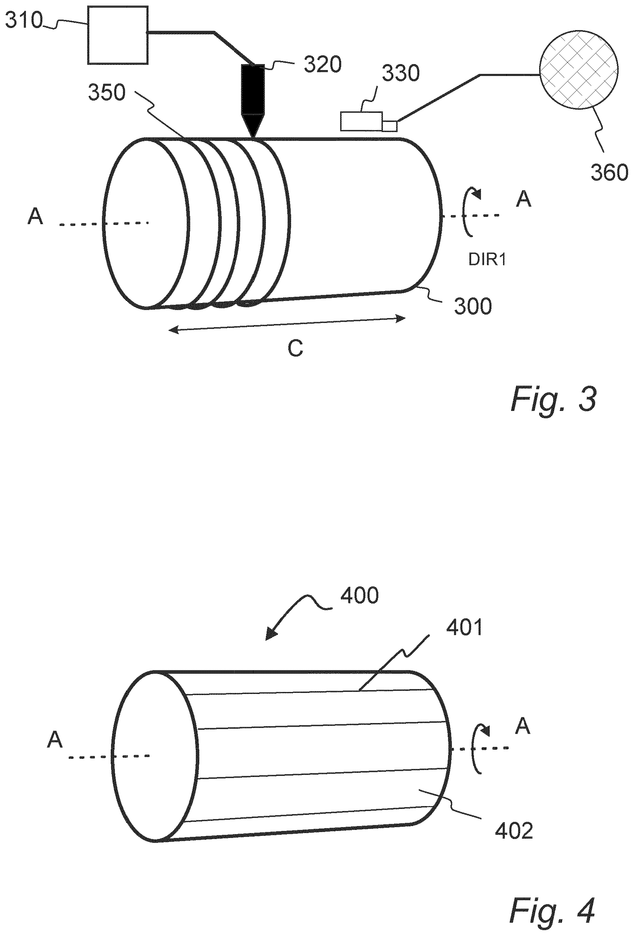

Figure 3 illustrates an apparatus for manufacturing natural fiber based raw wool according to an embodiment. A protein suspension 310 is fed to a nozzle 320. The refined fibrils of the protein suspension align in a nozzle. The aqueous suspension of water and fibrils of the protein suspension form an oriented fibril network in a nozzle 320.

The protein suspension 310 is directed onto a cylinder 300 via the nozzle 320. The protein suspension exiting the nozzle 320 is shaped according to the nozzle outlet opening. The nozzle outlet opening may be shaped as a round or as elliptic, for example. The protein suspension exits the nozzle 320 in a form of a continuous strand having a cross-sectional shape corresponding to that of the nozzle outlet opening. The protein suspension exiting the nozzle 320 may have round or elliptic cross-sectional shape.

The nozzle 320 may be arranged to move back and forth along the longitudinal direction C, i.e. direction of the rotational axis A, of the cylinder 300, along horizontal plane. The protein suspension 310 is directed to a certain horizontal level along the longitudinal cylinder surface. Due to rotation of the cylinder 300 and movement of the nozzle 320, the fed protein suspension 310 circles around the cylinder surface forming a round next to a round, or partly overlapping with adjacent rounds. The continuously injected protein suspension 310 moves on the surface of the cylinder 300 with the rotating cylinder 300. While the cylinder 300 rotates around its rotational axis A, the nozzle 320 is arranged to move to an adjacent place along longitudinal cylinder surface C.

Alternatively, two or more nozzles may be arranged adjacent, parallel along longitudinal cylinder surface C. The two or more nozzles are united, forming an integrated unit, and arranged to move concurrent along longitudinal cylinder surface C. The distance between two adjacent nozzles may be in order of centimeters, for example 1 cm. The nozzle(s) are oscillating along the longitudinal dimension of the cylinder. In case of two or more nozzles arranged at 1 cm distance from each other, the time that the nozzles take to move 1 cm corresponds to drying time of the protein suspension injected on the cylinder surface. Moving speed of nozzles is arranged such that time for a length of the dimension between the two adjacent nozzles is arranged to correspond to drying time of the protein suspension.

During drying water is removed and fibrils star forming hydrogen bonds. Thereby fiber is formed. Dried protein suspension or fiber forming or fiber refer to dry content of at least 70 wt-%. Fiber yarn count comprises 1-20 dtex. Fiber diameter may be 15-70 µm. Fiber tensile strength may be 10-25 cN/tex, preferably 15-20 cN/tex, and comprise stretch or elongation break ofl0-30%, preferably 15-25%.

In case of multiple nozzles along the longitudinal dimension of the cylinder, the multiple nozzles may be placed next to each other along the whole longitudinal dimension of the cylinder. Thus, there is no need for movement of nozzles, but those may be fixed in their places. In this case the drying time of the protein suspension corresponds to time of rotation of the cylinder.

With two or more nozzles, the cylinder is covered with protein suspension and fiber is formed faster than in case of one nozzle. Accordingly extracting fibers, providing oil or wax and other relating functions shall be accomplished at corresponding pace.

The protein suspension exit from a nozzle and/or injection onto a surface may be controlled hydraulically or pneumatically. Velocity of the protein suspension exiting the nozzle may be controlled by pressure applied on the protein suspension at the nozzle.

An oil or wax supply 330 may be arranged relative to a surface of the cylinder 300. The oil or wax supply 330 is arranged to move, in an oscillating manner, as the nozzle 320, along longitudinal dimension of the cylinder 300, parallel with the axis of rotation A of the cylinder 300, at certain vertical level. In case of oscillating nozzle(s) 320 the oil or wax supply 330 is arranged to oscillate simultaneously with the nozzle(s) 320.

Rotation of a cylinder may be controlled externally, for example by an electric motor, whose rotational speed is adjustable. A cylinder or a curved belt may generate a centripetal acceleration of 1-1000 g, preferably 100-500 g. Diameter of a cylinder may be 1-6 m. Rotational speed of the surface of the cylinder may be 5-25 m/s. The centripetal force (Fcp) acting on suspension (m) is dependent on radius (r) of the cylinder and its rotational surface speed (v). The centripetal force (Fcp) acting on suspension (m) is dependent on radius (r) of curvature of a belt and its rotational surface speed (v). Mathematically: Fcp=ma=mv2/r; whrein a=v2/r.

The protein suspension is dried on the surface of the cylinder 300. This may be effected internally and/or externally. Heating internally may be effected via electric heating resistor, heating steam or air. Heating externally may be effected via irradiation, heating and/or air blow. The dried protein suspension forms a fiber 350 onto surface of the cylinder 300. The fiber 350 is extracted from the surface of the cylinder 300. Extracting may be based on blowing, suction, vacumization, scraping or dropping fibers from the surface based on gravitation. The fiber 350 may be extracted mechanically or using vacuum or pressurized air. The extraction may be implemented manually or automatically. An extractor may be placed on a side of the cylinder 300 opposite to the nozzle 320. The extractor may have a fixed place, or the extractor may be arranged to move, in an oscillating manner, as the nozzle 320, along longitudinal dimension of the cylinder 300, parallel with the axis of rotation A of the cylinder 300, at certain vertical level. The extractor is arranged to oscillate simultaneously with the nozzle. The extractor may be integrated with the oil or wax supply 330 and move with it. Oil or wax is supplied on a cylinder surface after the fiber has been extracted from the cylinder surface.

The extracted fiber 360 is in form of unoriented and entangled fiber based raw wool, which may comprise uneven clumps among fluffy fiber based raw wool. When the fiber based raw wool comprises continuous fiber, the length of fiber is arranged to be cut or shortened to form staple fibers. After shortening fiber based raw wool comprising staple fibers is formed.

The fiber comprises linear mass density of 1-20 dtex, which relates to an amount of mass per unit length (1 tex = 1 g/1000 m; and 1 decitex =1 dtex =1 g/ 10000 m). Tenacity of the fiber comprises 10-25 cN/tex, preferably 15-20 cN/tex. Stretch to break the fiber is10-30%, preferably 15-25%. Some of the oil from the drying surface is present in the fiber surface. The oil or wax on the fiber surface has effect on further processing of the fiber based raw wool, for example to the friction and adhesion between fibers of the raw based wool.

Figure 4 illustrates a cylinder as an example of a surface according to an embodiment of the invention. Figure 4 shows axis of rotation A of the cylinder 400. Cylinder may comprise an even, flat external surface or a grooved surface, as illustrated in the Figure 4. Grooves 401 are arranged onto external surface of the cylinder 400 parallel with the axis of rotation A of the cylinder 400. The injected protein suspension is in contact with ridges 402 between the grooves 401 of the cylinder surface. Grooves 401 form a weak point for the protein suspension and have effect of forming a discontinuity for the continuously injected protein suspension. The continuously injected protein suspension has a break at each groove. Thereby the protein suspension forms separate parts of length of ridges 402 between the grooves 401. After extensive water is dried, staple fiber is formed onto the cylinder, on the ridges 402 between the grooves 401. Length of formed staple fibers is determined by the length between grooves 401 on the cylinder surface.

In case of grooved cylinder, the fiber based raw wool extracted from the cylinder comprises staple fibers. No additional refining, shorting or cutting means or phases are needed. The staple fiber based raw wool is processable. The fiber based raw wool comprises staple fibers, which have been shortened to a predefined length with the aid of grooves. The spacing between the grooves on the cylinder surface determine length of the staple fibers.

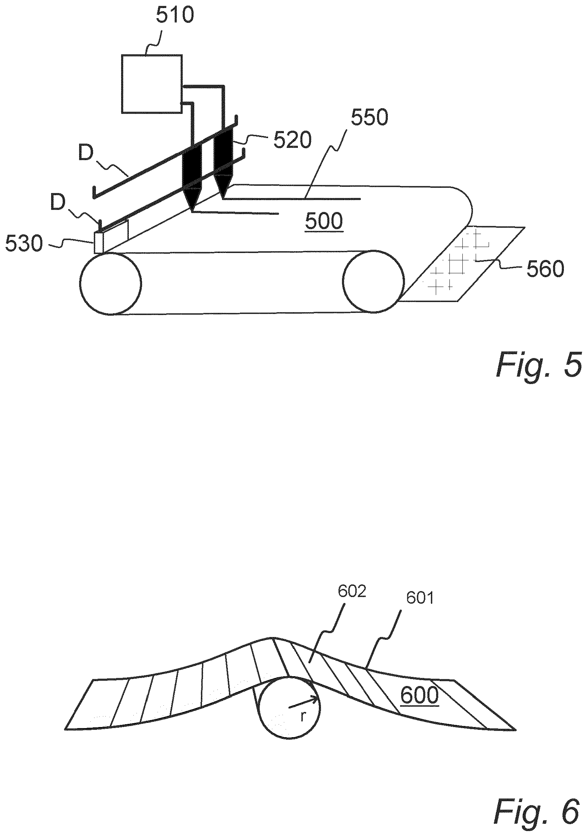

Figure 5 illustrates an apparatus for manufacturing natural fiber based raw wool according to an embodiment. A protein suspension 510 is fed to a one or more nozzle 520. The refined fibrils of the protein suspension align in a nozzle. The fibrils of the protein suspension form an oriented fibril network in a nozzle 520.

The protein suspension 510 is directed onto a belt 500 via the nozzle(s) 520. The protein suspension exiting the nozzle 520 is shaped according to the nozzle outlet opening. The nozzle outlet opening may be shaped as a round or as elliptic, for example. The protein suspension exits the nozzle 520 in a form of a continuous strand having a cross-sectional shape corresponding to that of the nozzle outlet opening. The protein suspension exiting the nozzle 520 may have round or elliptic cross-sectional shape.

Two or more nozzles may be arranged adjacent, parallel along cross- sectional or transverse belt surface. Cross/transverse dimension refers to a width dimension of the belt; perpendicular to a longitudinal dimension of the belt, which corresponds to the moving direction of the belt. The two or more nozzles may be united, form an integrated unit. The distance between two adjacent nozzles may be in order of 0.5-50 mm, or 0.5-20 mm, for example 1 mm. The multiple nozzles may be placed next to each other along the whole cross dimension of the belt. In such case the whole cross dimension of the belt is covered with injected protein suspension at the same time via multiple nozzles.

During drying water is removed and fibrils star forming hydrogen bonds. Thereby fiber is formed. Hydrogen bonds are formed when dry content is from 70 wt-% to 100 wt-%. Dried protein suspension or fiber forming or fiber refer to dry content of at least 70 wt-%.

The protein suspension exit from a nozzle and/or injection onto a surface may be controlled hydraulically or pneumatically. Velocity of the protein suspension exiting the nozzle may be controlled by pressure applied on the protein suspension at the nozzle.

An oil or wax supply 530 may be placed before the nozzle(s) 520 in relation to the moving direction of the belt 500. The oil or wax supply 530 may be arranged to move, in an oscillating manner along cross dimension of the belt 500, perpendicular with direction of movement of the belt 500. The oil or wax supply 530 is arranged after fiber extraction phase. Oil or wax is supplied on the surface of the belt 500. The protein suspension is injected on an oily belt surface.

The protein suspension is dried on the surface of the belt 500. This may be effected internally and/or externally. Heating internally may be effected via the belt, e.g. by an electric heating resistor, heating steam or air. Heating externally may be effected via irradiation, heating and/or air blow. The dried protein suspension forms a fiber 550 onto surface of the belt 500. The fiber 550 is extracted from the surface of the belt 500. Extracting may be based on blowing, suction, vacumization, scraping or dropping fibers from the surface based on gravitation. The fiber 550 may be extracted mechanically or using vacuum or pressurized air. The extraction may be implemented manually or automatically. An extractor may be placed on any part of the belt 500, where the dryness of the fiber is at a desired level, for example over 70 wt-%. The extractor may be placed at end of the belt, or at either external side of the belt, wherein upper external side of the belt is arranged to move at opposite direction than the lower external side of the belt. It is possible to convey protein suspension or fiber on another belt for further drying.

Oil or wax is supplied on a belt surface before the protein suspension is injected onto the surface.

The extracted fiber 560 is in form of unoriented and entangled fiber based raw wool, which may comprise uneven clumps among fluffy fiber based raw wool. When the fiber based raw wool comprises continuous fiber, the length of fiber is arranged to be cut, shortened or refined to form staple fibers. After shortening fiber based raw wool comprising staple fibers is formed.

The fiber comprises linear mass density of 1-20 dtex, which relates to an amount of mass per unit length (1 tex = 1 g/1000 m; and 1 decitex =1 dtex =1 g/ 10000 m). Tenacity of the fiber comprises 10-25 cN/tex, preferably 15-20 cN/tex. Stretch to break or elongation break of the fiber comprises 10-30%, preferably 15-25%. Some of the oil or wax from the drying surface is present in the fiber surface. The oil or wax on the fiber surface has effect on further processing of the fiber based raw wool, for example to the friction and adhesion between fibers of the raw based wool.

Figure 6 illustrates a belt as an example of a surface according to an embodiment of the invention. Figure 6 shows belt 600 comprising curved and grooved surface. Figure 6 shows a belt for forming staple fibers, while in case of even, flat, non-curved belt a continuous fiber is formed on the belt. The curved surface of the belt comprises a radius of curvature of 0.25-4 m.

Grooves 601 are arranged onto external surface of the belt 600. The grooves 601 are arranged along a transverse direction of the belt 600, perpendicular to the longitudinal dimension or direction of movement of the belt 600. The injected protein suspension is in contact with ridges 602 between the grooves 601 of the belt surface. Grooves 601 form a weak point for the protein suspension and have effect of forming a discontinuity for the continuously injected protein suspension. The continuously injected protein suspension has a break at each groove. Thereby the protein suspension forms separate parts of length of ridges 602 between the grooves 601. After extensive water is dried, staple fibers are formed onto the belt, on the ridges 602 between the grooves 601. Length of formed staple fibers is determined by the length between grooves 601 on the belt surface.

In case of grooved belt, the fiber based raw wool extracted from the belt comprises staple fibers. No additional refining, shorting or cutting means or phases are needed. The staple fiber based raw wool is processable. The fiber based raw wool comprises staple fibers, which have been shortened to a predefined length with the aid of grooves. The spacing between the grooves on the belt surface determine length of the staple fibers.

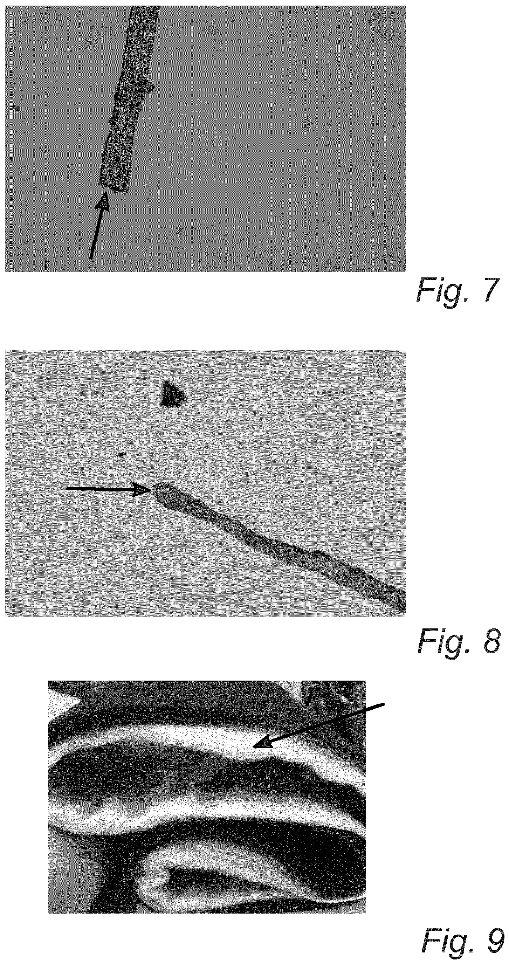

When the continuous fiber is shortened as staple fibers, cut end of a staple fiber is sharp, or at least substantially sharp. Sharp cut end is illustrated in a Figure 7. When staple fiber is formed on a curved, grooved surface by the grooves, the end of the staple fiber is uneven or irregular in comparison to the sharp cut end of a staple fiber. Figure 8 shows end of a staple fiber, which has been formed by a curved, grooved surface.

Groove causes interruption to the applied continuous protein suspension, thereby forming separated portions of certain length, which, after dried, form staple fibers. Such interrupted surface of a staple fiber may show fibrils or smaller portions. In an extended view, uneven end surface of interruption shows irregular shape.

Figure 9 shows a fiber based raw wool arranged on a conveyor belt. Staple fibers are extracted from cylinder to a moving conveyor belt where they are arranged in a random, bulky network.

The surface, of e.g. a belt or a cylinder, may comprise hydrophobic surface material. External surface of a cylinder or a belt may be covered with a nonporous hydrophobic polymer coating. The polymer coating has effect of forming a hydrophobic surface. The hydrophobic polymer coating may be covered with oil or wax. The coating and oil or wax coverage of the surface enable to achieve a hydrophobic, low friction and low contact angle hysteresis external surface. The oil or wax effects on protein suspension remaining in contact with the external surface via the oil or wax. The polymer coating and oil or wax on the surface and/or centripetal force of a rotating cylinder enable controlling and maintaining round cross-sectional shape of the protein suspension injected on the surface. Oil or wax and low contact angle hysteresis on the outer surface of the cylinder or the belt have effect on maintaining the round cross-sectional shape of the protein suspension during drying. Oil has effect of reducing risk of the protein-solution adhering to the surface. Oil or wax has effect of providing stable cross-sectional shape fiber and/or avoid formation of unwanted wide, thin and/or weak ribbons on the surface. Part of the oil or wax remains with the fiber and acts as finishing agent. Oil may be used for example to control friction between fibers and/or between fibers and metal during processing. These are desired parameters for processing the fiber based raw wool during further processing and formation of products, like yarn, non-woven or other.

The protein suspension directed through a nozzle onto a surface is dried on the surface. Drying is accomplished via heat and, in case of a cylinder, a rotating motion of a cylinder. Rotation of the cylinder and/or heating enable drying of provided protein suspension on the surface. A continuous fiber may be formed on the surface. Alternatively, stable fibers may be formed on the surface. The protein suspension may be dried externally and/or internally via the surface of a cylinder or of a belt. The cylinder or the belt may comprise a heating element. The cylinder or the belt may comprise an internal heater. The internal heater may implement heating electronically, via a resistor, or via hot steam. The cylinder or the belt may comprise an external heater. The external heater may provide irradiation or air blow or steam blow towards the surface in order to dry the protein suspension on the surface. The heater(s) enable drying protein suspension applied on the surface. Heater or heating elements are arranged to dry the protein suspension by removing water.

Oil or wax is applied on the surface. Oil or wax on the surface has effect of reducing surface tension, friction and/or surface contact angle hysteresis. Oil or wax may comprise, but is not limited to, fiber finishing oils to reduce fiber-fiber friction or fiber-metal friction, or a vegetable oil or a non-immiscible fluid.

The surface is arranged to move and convey the injected protein suspension. The surface may comprise grooves. Staple fibers may be formed on a curved and grooved surface. The surface may comprise a curver or round cylinder surface, or a curved belt surface. The grooves are aligned transverse to the direction of movement of a surface. Grooves are arranged transverse to the direction of movement of a belt. The grooves extend along transverse direction of the belt, which is perpendicular to the longitudinal dimension, or direction of movement of the belt. Grooves are arranged transverse to the direction of rotation of a cylinder. The grooves extend along length direction of the cylinder, being in parallel with the axis of rotation of the cylinder. The surface comprises thin grooves, in comparison to wide ridges between the grooves. The ridges of the surface form support surface for injected protein suspension, while grooves form a discontinuation places. Grooves and the ridges between them enable forming staple fibers on the surface. Injected protein suspension is arranged to break at a groove. Spacing between the grooves is configured to define length of formed staple fibers. Width of the grooves may be 0.5-5 mm, preferably 2-3 mm. Depth of the grooves may be 0.5-10 mm, preferably 2-3 mm.

Centripetal force of a moving surface, hydrophobic properties of a surface and oil properties have effect on preserving round cross section of the injected protein suspension and fiber, as its dried form, on the surface.

As free water is removed from the protein suspension during drying, hydrogen bonds start to appear.

The dried protein solution forms a continuous fiber (strand) onto a continuous surface. Grooved and curved surface enable forming of stable fibers without additional refining or shortening after drying. The staple fibers comprise length of 6-80 mm, preferably 30-70 mm.

Long fibers or staple fibers may be interlocked together in order to form a permanent network of fibers. Disintegration of the hydrogen bonds, may be done by exposing the staple fibers to water or aqueous solution. A minor mechanical or hydrodynamical force, like a pull or a twist or a hydrodynamic shear, disintegrates a wetted staple fiber composition or product. When exposed to water, the staple fibers will return into separate primary protein fibrils. This enables forming water disposable products.

The natural fiber based raw wool comprises large specific surface area and low density. It provides good filtering properties and a good insulator, while it's thermal conductivity is low. Due to uneven fiber surface, the natural fiber based raw wool comprises high friction, which is desired property for further processing, for example manufacturing non-woven material.

The fiber based raw wool may be processed as a raw wool sheared from a sheep. The fiber based raw wool comprises staple fibers. The fiber based raw wool comprises staple fibers in fluffy arrangement, in unorganized, unoriented order and forming clumps or conglomerates of different densities.

The fiber based raw wool may be carded. Generally carding orients, detangles and cleans raw wool towards oriented strands. Carding may be performed by a card, by a carding machine, by heckling machine. Carding machine may have surface covered with carding clothing or soft-bristled brush attachment. During carding the staple fibers orientate towards common fiber orientation and the staple fiber density becomes more even, while reducing clumps. Due to carding fibers tend to orient similarly, thereby having substantially similar orientation among longitudinal dimension of fibers. The carded fiber based raw wool comprises at least mainly oriented staple fibers and density variations or clumps are reduced. The structure tends to become more homogenous during carding.

Natural fiber based raw wool has properties desired during carding, like low friction between fibers and/or between fibers and metal (of cards).

The carded fiber based raw wool may be processed as a yarn or as a non- woven material. Yarn may be made from carded fiber based raw wool by forming a continuous pre-yarn and spinning several pre-yarns as a yarn. The yarn may be used for manufacturing textiles of different kind. A textile may be made of yarn using known textile manufacturing processes and equipment.

A non-woven material may be produced from the carded fiber based raw wool by a non-woven process, or felting, for example needle punch, hydro-entanglement or other suitable method. The non-woven process binds the staple fibers mechanically in order to produce continuous non-woven material or fabric. Adhesive may be added in order to enhance bonding.

Non-woven material may be used to manufacture hygiene products. The hygiene products may be disposed with water, whereby the product breaks down to protein fibrils. The hygiene products may comprise flushable products, which disintegrate into water. Hygiene products may comprise wipes or diapers. The products made of the staple fiber based raw wool have firm fabric and feel, when those are dry. However, underlying fibrils are small and locked together via hydrogen bonds, which become very weak at aqueous environment. Once in water, even a low shear will cause staple fibers to disintegrate back to sorter protein fibrils. In addition to disposability, the non-woven material made of staple fiber based raw wool has ability to absorb and retain water. This ability is desired for products like diapers and alike.

Yarn made of natural fiber based raw wool may have a yarn count of 5-200 tex. The yarn comprises tenacity of 5-15 cN/tex; and elongation at break of 3-10 %. Non-woven material made of natural fiber based raw wool comprises a density of 10-100 kg/m3.

The manufacturing process enables providing yarn and/or non-woven material in an economically and environmentally friendly way. The provided fiber based raw wool is provided by compact manufacturing phase, even as a single process. The fiber based raw wool is processable with process and equipment known from raw wool processing and handling. The fiber based raw wool may be processed as yarn using yarn spinning equipment or as non-woven material using non-woven process and method.

The staple fiber based raw wool or products have effect on biodegradability. Discarding is ecological and use of natural based cellulosic fibers enables recycling and reuse.

A natural fiber based raw wool may be used as an insulator. It forms a usable insulator before or after carding.

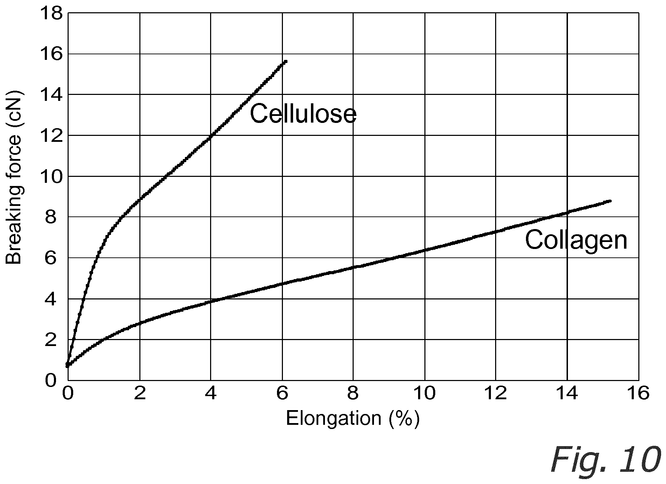

Figure 10 illustrates an example of fiber strength/breaking force (cN) and elongation (%) of collagen and cellulose respectively. Breaking force of collagen is less than cellulose, but the elongation and stretch is much higher for collagen.

Fig. 10 shows an example of the mechanical properties of a collagen based staple fiber product and a cellulose staple fiber product. Staple fiber product referred to as "cellulose" comprises 85% of fibrous elements of natural cellulose and 15 % of additive(s). Staple fiber product referred to as "collagen" comprises 85% of fibrous elements of collagen and 15 % of additive(s). Linear mass density of the fiber products is 7.5 dtex. Elongation of the cellulose staple fiber product is about 6%. Elongation of the fiber product of collagen is about 15%. Breaking force of the fiber product of cellulose is above 14 cN. Breaking force of the fiber product of collagen is above 8 cN.

## Claims

1. A method for manufacturing a staple fiber based on natural protein fiber, the method comprising the steps of
- providing a protein suspension,
- the protein suspension comprising fibrils of said natural protein fiber,
- directing the protein suspension through a nozzle (520) onto a surface (500),
- drying the protein suspension on the surface to a dry concentration of at least 70% by weight for forming a protein based fiber,
- extracting the fiber from the surface and providing the staple fiber.

2. A method according to claim 1, wherein natural protein fibers comprise collagen fibers.

3. A method according to any of the previous claims, comprising applying oil or wax to the surface.

4. A method according to any of the previous claims, comprising shortening the fiber extracted from the drying surface in order to form staple fibers.

5. A method according to any of the previous claims, comprising shortening the protein suspension to predetermined length(s) on a curved drying surface comprising grooves or ridges in order to form staple fibers.

6. A method according to any of the previous claims wherein the collagen suspension comprises collagen fibrils of naturals fibers and wherein the collagen content of the suspension is more than 50% of the total weight of natural protein fiber fibrils in the suspension.

7. A method according to any of the previous claims wherein the protein suspension comprises fibrils of natural fibers and wherein the collagen protein content of the suspension is more than 60% of the total weight of natural protein fiber fibrils in the suspension.

8. A method according to any of the previous claims wherein the protein suspension further comprises additive(s) including rheology modifier(s), binder(s), cation active reagent(s), crosslinking agent(s), dispersion agent(s), pigment(s), and/or other modifier(s).

9. An apparatus for manufacturing a staple fiber based on natural protein fiber comprising
- a nozzle arranged to direct a protein suspension onto a surface,
- the protein suspension comprising fibrils of said natural protein fiber,
- a dryer arranged to dry the protein suspension on the surface for forming a fiber,
- an extractor arranged to extract the fiber from the surface and an arrangement to form the staple fiber.

10. An apparatus according to claim 9, wherein the protein suspension is directed to the surface and comprises a cross- sectional shape corresponding to a shape of a nozzle outlet opening, optionally a round or an elliptic cross-sectional shape.

11. An apparatus according to any of the claims 9-10, wherein the fiber comprises dry concentration of at least 70 wt-%.

12. A fiber based raw wool comprising staple fibers provided according to any of the claims 1-8, wherein the staple fibers reconstructed on the basis of protein fibrils are mechanically subdivided from natural protein fibers, wherein the protein fibrils are interlocked by hydrogen bonds and the fiber based raw wool comprises unoriented, entangled, fluffy network of staple fibers.

13. Use of the staple fiber according to any of the previous claims 1-8 in textile and/or shoe industry.

## Patentansprüche

1. Verfahren zum Herstellen einer Stapelfaser auf der Basis von natürlicher Proteinfaser, wobei das Verfahren die Schritte umfasst
- Bereitstellen einer Proteinsuspension,
- wobei die Proteinsuspension Fibrillen der natürlichen Proteinfaser umfasst,
- Richten der Proteinsuspension durch eine Düse (520) auf eine Oberfläche (500),
- Trocknen der Proteinsuspension auf der Oberfläche auf eine Trockenkonzentration von mindestens 70 Gew.-% zum Bilden einer proteinbasierten Faser,
- Extrahieren der Faser von der Oberfläche und Bereitstellen der Stapelfaser.

2. Verfahren nach Anspruch 1, wobei natürlichen Proteinfasern Kollagenfasern umfassen.

3. Verfahren nach einem der vorstehenden Ansprüche, umfassend das Aufbringen von Öl oder Wachs auf die Oberfläche.

4. Verfahren nach einem der vorstehenden Ansprüche, umfassend das Verkürzen der aus der Trocknungsoberfläche extrahierten Faser, um Stapelfasern zu bilden.

5. Verfahren nach einem der vorstehenden Ansprüche, umfassend das Verkürzen der Proteinsuspension auf eine bestimmte Länge bzw. auf bestimmte Längen auf einer gekrümmten Trocknungsoberfläche, die Rillen oder Rippen umfasst, um Stapelfasern zu bilden.

6. Verfahren nach einem der vorstehenden Ansprüche, wobei die Kollagensuspension Kollagenfibrillen natürlicher Fasern umfasst und wobei der Kollagengehalt der Suspension mehr als 50 % des Gesamtgewichts der natürlichen Proteinfaserfibrillen in der Suspension beträgt.

7. Verfahren nach einem der vorstehenden Ansprüche, wobei die Proteinsuspension Fibrillen von Naturfasern umfasst und wobei der Kollagenproteingehalt der Suspension mehr als 60 % des Gesamtgewichts der natürlichen Proteinfaserfibrillen in der Suspension beträgt.

8. Verfahren nach einem der vorstehenden Ansprüche, wobei die Proteinsuspension ferner ein Additiv bzw. Additive umfasst, einschließlich eines Rheologiemodifikators bzw. Rheologiemodifikatoren, eines Bindemittels bzw. Bindemitteln, eines kationenaktiven Reagenzes bzw. kationenaktiven Reagenzien, eines Vernetzungsmittels bzw. Vernetzungsmitteln, eines Dispersionsmittels bzw. Dispersionsmitteln, eines Pigments bzw. Pigmente und/oder eines anderen Modifikators bzw. Modifikatoren umfasst.

9. Vorrichtung zum Herstellen einer Stapelfaser basierend auf natürlicher Proteinfaser, umfassend
- eine Düse, die angeordnet ist, um eine Proteinsuspension auf eine Oberfläche zu lenken,
- wobei die Proteinsuspension Fibrillen der natürlichen Proteinfaser umfasst,
- einen Trockner, der angeordnet ist, um die Proteinsuspension auf der Oberfläche zum Bilden einer Faser zu trocknen,
- einen Extraktor, der angeordnet ist, um die Faser von der Oberfläche zu extrahieren und eine Anordnung, um die Stapelfaser zu bilden.

10. Vorrichtung nach Anspruch 9, wobei die Proteinsuspension auf die Oberfläche gerichtet ist und eine Querschnittsform umfasst, die einer Form einer Düsenauslassöffnung entspricht, optional einer runden oder einer elliptischen Querschnittsform.

11. Vorrichtung nach einem der Ansprüche 9-10, wobei die Faser eine Trockenkonzentration von mindestens 70 Gew.-% umfasst.

12. Rohwolle auf Faserbasis, umfassend Stapelfasern, die nach einem der Ansprüche 1-8 bereitgestellt sind, wobei die Stapelfasern, die auf der Basis von Proteinfibrillen rekonstruiert werden, mechanisch von natürlichen Proteinfasern unterteilt sind, wobei die Proteinfibrillen durch Wasserstoffbrückenbindungen miteinander verbunden sind und die Rohwolle auf Faserbasis ein unorientiertes, verflochtenes, flauschiges Netzwerk von Stapelfasern umfasst.

13. Verwendung der Stapelfaser nach einem der vorstehenden Ansprüche 1-8 in der Textil- und/oder Schuhindustrie.

## Revendications

1. Procédé de fabrication d'une fibre discontinue à base de fibre de protéine naturelle, le procédé comprenant les étapes suivantes
- la fourniture d'une suspension protéique,
- la suspension protéique comprenant des fibrilles de ladite fibre de protéine naturelle,
- la direction de la suspension de protéines à travers une buse (520) sur une surface (500),
- le séchage de la suspension protéique sur la surface à une concentration sèche d'au moins 70 % en poids pour former une fibre à base de protéines,
- l'extraction de la fibre de la surface et fournir la fibre discontinue.

2. Procédé selon la revendication 1, dans lequel les fibres de protéine naturelle comprennent des fibres de collagène.

3. Procédé selon l'une quelconque des revendications précédentes, comprenant l'application d'huile ou de cire à la surface.

4. Procédé selon l'une quelconque des revendications précédentes, comprenant le raccourcissement de la fibre extraite de la surface de séchage pour former des fibres discontinues.

5. Procédé selon l'une quelconque des revendications précédentes, comprenant le raccourcissement de la suspension protéique à une ou des longueur(s) prédéterminée(s) sur une surface de séchage incurvée comprenant des rainures ou des crêtes afin de former des fibres discontinues.

6. Procédé selon l'une quelconque des revendications précédentes, dans lequel la suspension de collagène comprend des fibrilles de collagène de fibres naturelles et dans lequel la teneur en collagène de la suspension est supérieure à 50 % du poids total de fibrilles de fibres de protéines naturelles dans la suspension.

7. Procédé selon l'une quelconque des revendications précédentes, dans lequel la suspension de protéines comprend des fibrilles de fibres naturelles et dans lequel la teneur en protéines de collagène de la suspension est supérieure à 60 % du poids total de fibrilles de fibres de protéines naturelles dans la suspension.

8. Procédé selon l'une quelconque des revendications précédentes, dans lequel la suspension protéique comprend en outre un ou des additif(s) incluant un ou des agent(s) modifiant la rhéologie, un ou des liant(s), un ou des réactif(s) actif(s) cationique(s), un ou des agent(s) de réticulation, un ou des agent(s) de dispersion, un ou des pigment(s), et/ou un autre(s) agent(s) modifiant(s).

9. Appareil pour fabriquer une fibre discontinue à base de fibre de protéine naturelle comprenant
- une buse agencée pour diriger une suspension protéique sur une surface,
- la suspension protéique comprenant des fibrilles de ladite fibre de protéine naturelle,
- un sécheur agencé pour sécher la suspension protéique sur la surface pour former une fibre,
- un extracteur agencé pour extraire la fibre de la surface et un agencement pour former la fibre discontinue.

10. Appareil selon la revendication 9, dans lequel la suspension protéique est dirigée vers la surface et comprend une forme en coupe transversale correspondent à une forme d'une ouverture de sortie de buse, éventuellement une forme ronde ou une forme en coupe transversale elliptique.

11. Appareil selon l'une quelconque des revendications 9 à 10, dans lequel la fibre comprend une concentration sèche d'au moins 70 % en poids.

12. Une laine brute à base de fibres comprenant des fibres discontinues fournies selon l'une quelconque des revendications 1 à 8, dans laquelle les fibres discontinues reconstruites sur la base des fibrilles de protéines sont divisées mécaniquement de fibres de protéines naturelles, dans lequel les fibrilles de protéines sont entrelacées par des liaisons hydrogène et la laine brute à base de fibres comprend un réseau pelucheux, enchevêtré, non orienté de fibres discontinues.

13. Utilisation de la fibre d'agrafes selon l'une quelconque des revendications précédentes 1 à 8 dans l'industrie textile et/ou chaussure.
